# EUROPÄISCHE PATENTSCHRIFT

(11) **EP 0 783 528 B1**
(45) Veröffentlichungstag und Bekanntmachung des Hinweises auf die Patenterteilung: **12.08.1998**
(21) Anmeldenummer: 95935380.6
(22) Anmeldetag: 26.09.1995
(51) Int. Cl.: C08B 30/12, A61K 31/715

(54) **VERFAHREN ZUR HERSTELLUNG VON STÄRKEABBAUPRODUKTEN**
METHOD OF PRODUCING STARCH DECOMPOSITION PRODUCTS
PROCEDE DE FABRICATION DE PRODUITS DE DECOMPOSITION DE L' AMIDON

(30) Priorität: 29.09.1994 DE 4434877
(43) Veröffentlichungstag der Anmeldung: 16.07.1997
(73) Patentinhaber: Fresenius AG, 61350 Bad Homburg v.d.H (DE)
(72) Erfinder: SOMMERMEYER, Klaus, D-61191 Rosbach v.d.H. (DE); GÖRG, Michael, D-61197 Florstadt (DE); HENNING, Klaus, D-61250 Usingen (DE)
(74) Vertreter: Luderschmidt, Schüler & Partner GbR
(86) Internationale Anmeldenummer: EP9503806
(87) Internationale Veröffentlichungsnummer: WO9610042

(56) Entgegenhaltungen:
- WO-A-93/21008
- DE-A- 2 216 854
- DE-A- 4 132 701
- DIE STÄRKE, Bd. 36, Nr. 4, 1984 DE, Seiten 116-116-121, F. MEUSER ET AL. 'Einsatz der Hochdruckhomogenisierung zur Stärkegewinnung aus Mais'
- DATABASE WPI Week 8837 Derwent Publications Ltd., London, GB; AN 88-261559 & JP,A,63 190 821 (DAICEL CHEM. IND. KK) , 8.August 1988

## Beschreibung

Die Erfindung betrifft ein Verfahren zur Herstellung von Stärkeabbauprodukten mit enger Molekulargewichtsverteilung durch Spaltung von Stärke oder Stärkederivaten.

Stärkeabbauprodukte, insbesondere Hydroxyethylstärke (HES), spielen eine wichtige Rolle in vielen Bereichen der Medizin, in der sie überwiegend als Plasmaexpander, jedoch auch bei der Dialyse (Peritonealdialyse) eingesetzt werden.

Daneben werden Stärkeabbauprodukte diätetisch verabreicht.

Zur Herstellung von HES werden bislang sowohl hydrolytisch als auch amylase-abgebaute Stärken eingesetzt. Ein enzymatisches Verfahren ist beispielsweise in der DE-C 33 13 600 offenbart.

Bei den vorstehend genannten chemischen oder biochemischen Verfahren entstehen Produkte mit breiter Molekulargewichtsverteilung mit einem beträchtlichen Anteil an niedermolekularen Verbindungen, wie Glucose, Maltose oder Oligosaccharide, wobei als Nebenprodukte beispielsweise Natriumchlorid oder die eingesetzten Enzyme anfallen. Diese unerwünschten Bestandteile müssen anschließend in weiteren Prozeßschritten entfernt werden, beispielsweise durch Fällung mit organischen Lösungsmitteln (Aceton, Isopropanol), oder durch Ultrafiltration, was zum einen kostenintensiv ist und zum anderen die Ausbeute schmälert. Derartige Reinigungsoperationen sind jedoch notwendig, da üblicherweise ein Produkt mit möglichst enger Molekulargewichtsverteilung und hohem Reinheitsgrad angestrebt wird.

Ein solches Verfahren ist aus der DE-A- 41 32 701 bekannt, bei dem Stärke oder Stärkederivate in einem wässrigen Gemisch (Dispersionen, Suspensionen oder Lösungen) mit Ultraschall behandelt werden. Hierdurch soll das gewünschte mittlere Molekulargewicht in Abhängigkeit von der Behandlungszeit und der Beschallungsintensität in gewünschter Höhe bei sehr enger Molekulargewichtsverteilung unter praktischer Abwesenheit unerwünschter niedermolekularer Bestandteile einstellbar sein.

Dieses Verfahren ist jedoch technisch sehr aufwendig und darüber hinaus mit einem hohen Energieaufwand bei der Spaltung der Stärke behaftet.

Aus der DE-A-33 04 775 ist ein Verfahren zur Depolymerisation von Polysacchariden bekannt, das nur bei Lösungen von Polysacchariden mit Helix-Struktur, nicht aber bei Lösungen von Polysacchariden mit Einzelketten-Struktur oder aggregierter Konformation einsetzbar ist. Im übrigen hat es sich herausgesteilt, daß die Ultraschall-Depolymerisation für eine industriell durchführbare Depolymerisation großer Ansätze nicht geeignet ist.

Gemäß der WO 93/21008 wird Stärke zur Modifikation der physikalischen Eigenschaften in einer Kammer (Kolbenvorrichtung) einer oder mehreren abrupten Druckänderungen unterworfen. Dieses Verfahren arbeitet unter Anwendung eines Pascalisators. Dabei wird lediglich ein statischer Druck auf die Probe oder Flüssigkeit ausgeübt. Bei dieser Verfahrensweise wird kein Molekulargewichtsabbau erzielt. Dieses Verfahren ist daher zur Erzielung eines Stärkeabbauproduktes mit enger Molekulargewichtsverteilung nicht geeignet.

Der Erfindung liegt daher die Aufgabe zugrunde, ein Verfahren der eingangs erwähnten Art zur Verfügung zu stellen, das technisch einfach ist und darüber hinaus einen relativ geringen Energieaufwand bei der Spaltung benötigt, wobei die Bildung unerwünschter niedermolekularer Anteile der Stärke möglichst vermieden wird und die Abbauprodukte mit möglichst hoher Ausbeute anfallen.

Diese Aufgabe wird erfindungsgemäß dadurch gelöst, daß man die Spaltung durch Hochdruckhomogenisation durchführt.

Das erfindungsgemäße Verfahren hat gegenüber der dem Fachmann bekannten Ultraschall-Technik den Vorteil, daß die einzusetzende Technik bei der Herstellung von Emulsionen oder Dispersionen vielfach verwendet wird und daher in den meisten Betrieben bereits vorhanden ist, so daß die Investitionskosten relativ gering sind. Darüber hinaus ist die Hochdruckhomogenisation weniger energieaufwendig als die Ultraschall-Technik.

Bei dem erfindungsgemäß eingesetzten Verfahren zur Homogenisation wird die Flüssigkeit mittels Hochdruck-Pumpeneinheiten durch ein Präzisions-Aufschlußventil gepreßt. Die benötigten Drücke (500- > 2000 bar) und Anzahl der Durchgänge hängen dabei vom Zielmolekulargewicht ab. In diesem Ventil wird eine Kavitationszone hoher Leistungsdichte durchlaufen, in der die hochmolekularen Ketten unter der Einwirkung großer örtlicher Zug-, Druckund Schubspannungen zerstört bzw. aufgebrochen werden. Erfindungsgemäß führt die o.g. Behandlung zu einem Zerschlagen der langkettigen Struktur der Stärke oder Stärkederivate.

Gegenüber dem Verfahren gemäß DE-A-33 04 775 weist das erfindungsgemäße Verfahren den Vorteil auf, daß überraschenderweise auch verzweigt-kettige Polysaccharide ohne Helix-Struktur abgebaut werden können, was bei dem bekannten Verfahren nicht der Fall ist.

Erfindungsgemäß ist es möglich, ein gewünschtes mittleres Molekulargewicht (Gewichtsmittel M_{w}) durch Variation des angelegten Drucks, der Anzahl der Behandlungen, der Temperatur, der Konzentration der Stärke oder der Stärkederivate und des pH-Wertes bei enger Molekulargewichtsverteilung bis herab zu Molekulargewichten von 100 000 Dalton unter Abwesenheit unerwünschter niedermolekularer Bestandteile einzustellen. Insofern ergeben sich Ausbeuten von nahezu 100%.

Darüber hinaus sind bei diesem Verfahren keine Zusätze von Säuren, wie dies beim säurehydrolytischen Abbau der Fall ist, oder von Enzymen im Falle eines enzymatischen Abbaus notwendig, so daß weitere Reinigungsschritte, wie die Fällung mit organischen Lösungsmitteln oder die Diafiltration minimiert werden.

Als Ausgangsmaterial werden vorzugsweise native Stärke, teilhydrolysierte Stärke oder Derivate daraus eingesetzt, wobei Stärke aus amylose-freiem bzw. amylosearmem (< 5% Amylose) Amylopektin besteht. Vorteilhafterweise werden als Stärke Mais-, Reis- und/oder Sorghumstärke eingesetzt.

Stärkederivate sind teilhydrolysierte Stärke- oder weitere Stärkederivate, beispielsweise ein Hydroxyalkyl- oder Alkoxyalkyl-Stärkederivat, insbesondere Hydroxyethylstärkederivat. Die teilhydrolysierten Stärken können durch Säurehydrolyse und/oder durch Enzymhydrolyse erhalten werden. Die erfindungsgemäß verwendeten Stärkederivate weisen vorzugsweise ein mittleres Molekulargewicht von > 200 000 Dalton auf.

Die Stärke oder die Stärkederivate können in Form wässriger Dispersionen, Suspensionen oder Lösungen eingesetzt werden. Dabei wird unter Suspension eine Dispersion von unlöslichen Stärketeilchen verstanden, die nich kolloid sind. Vorliegende Lösungen enthalten gelöste Stärke bzw. Stärkederivate in Wasser. Andererseits können aber auch kolloide Mischungen von Stärke oder Stärkederivaten in Wasser eingesetzt werden. Derartige wässrige Lösungen können nach der Verkleisterung 5 - 40 Gew.-% Stärke oder Stärkederivate enthalten, wobei letztere ein mittleres Molekulargewicht über 200.000 Dalton aufweisen sollen. Andererseits können 5 - 60 Gew.-% wässrige Suspensionen von Stärke oder Stärkederivaten oder eine durch Verkleisterung hergestellte Dispersion einer nativen Stärke, vorzugsweise in einer Menge von 5 - 60 Gew.-%, oder eine 5 - 40 Gew. %ige wässrige Dispersion einer teilhydrolysierten Stärke oder eines teilhydrolysierten Stärkederivates einsetzbar sein.

Dabei können Stärkederivate, die zuvor hydrolytisch oder enzymatisch gespaltet worden sind, ohne vorherige Isolierung in wässriger Dispersion bzw. Lösung der Hochdruckhomogenisation unterworfen werden.

Die Hochdruckhomogenisation kann in an sich bekannter Weise und mit hierfür geeigneten, handelsüblichen erhältlichen Geräten durchgeführt werden.

Die Homogenisierungsbedingungen sind üblicherweise abhängig von der Art des Ausgangsstoffs, der Art der Reaktionsmischung und insbesondere dem angestrebten Ergebnis, das durch das gewünschte mittlere Molekulargewicht des Stärkeendproduktes vorgegeben ist.

Die Homogenisation wird üblicherweise bei einer Temperatur von 5 - 95°C durchgeführt, wobei die Raumtemperatur von etwa 20°C bevorzugt ist.

Die Homogenisation wird bei Drücken von 500 - 2000 bar durchgeführt, wobei aber auch über dem Grenzdruck von 2000 bar liegende Drücke zum Einsatz kommen können.

Üblicherweise wird nach mehreren Druckbehandlungen (Durchgängen) im Hochdruckhomogenisator ein Sättigungsniveau erreicht, das nicht mehr unterschritten wird. Dabei wird unter Durchgang ein einmaliges Durchpressen des Versuchsgutes durch den Spalt verstanden. Es ist der Abbau des Stärkepolymeren umso größer, je höher der Druck ist, d.h. mit wachsendem Druck werden kleinere, mittlere Molekulargewichte erhalten. Der Vorteil des erfindungsgemäßen Verfahrens ist dabei, daß sich ein Molekulargewicht relativ leicht durch die zuvor ermittelte Zahl der Durchgänge einstellen läßt gegenüber der Säurehydrolyse, die permanent viskosimetrisch überwacht werden muß.

Somit ist es durch die Wahl der Bedingungen relativ leicht möglich, ein mittleres Zielmolekulargewicht ohne aufwendige Viskositätsüberwachung zu erreichen.

Die nach dem erfindungsgemäßen Verfahren hergestellten Stärkeabbauprodukte können vorteilhafterweise zur Herstellung von verätherter oder veresterter Stärke (HES bzw. Acetylstärke) mit hohen Ausbeuten eingesetzt werden. Diese Stärkeabbauprodukte (insbesondere HES), finden medizinische Anwendung, besonders im Bereich der Volumenersatzmittel.

Weiterhin ist Gegenstand der Erfindung die Verwendung der nach dem erfindungsgemäßen Verfahren hergestellten Stärkeabbauprodukte für pharmazeutische Zusammensetzung für klinische, vorzugsweise parenterale Anwendungen. Des weiteren können die nach dem erfindungsgemäßen Verfahren hergestellten Stärkeabbauprodukte auch für pharmazeutische Zusammensetzungen verwendet werden, die in der Peritonealdialyse oder bei Blutplasmaersatzmitteln zur Anwendung kommen.

Sofern die erfindungsgemäßen Abbauprodukte von niedermolekularen Bestandteilen befreit werden sollen, die aus vorangegangenen Prozeßschritten stammen, können diese natürlich mittels der an sich bekannten Diafiltration durch geeignete Wahl von Membranen entfernt werden. Schließlich können die üblicherweise in Lösung befindlichen Stärkeabbauprodukte auch in den getrockneten Zustand mittels üblicher Verfahren (Konzentrierung der Lösung im Vakuum bzw. Lyophilisation und anschließende Sprühtrocknung) überführt werden.

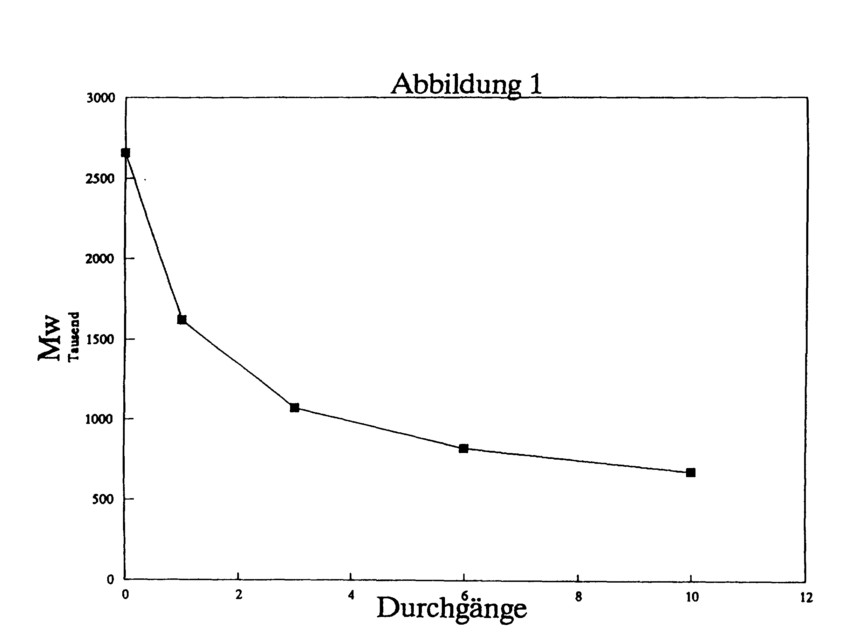

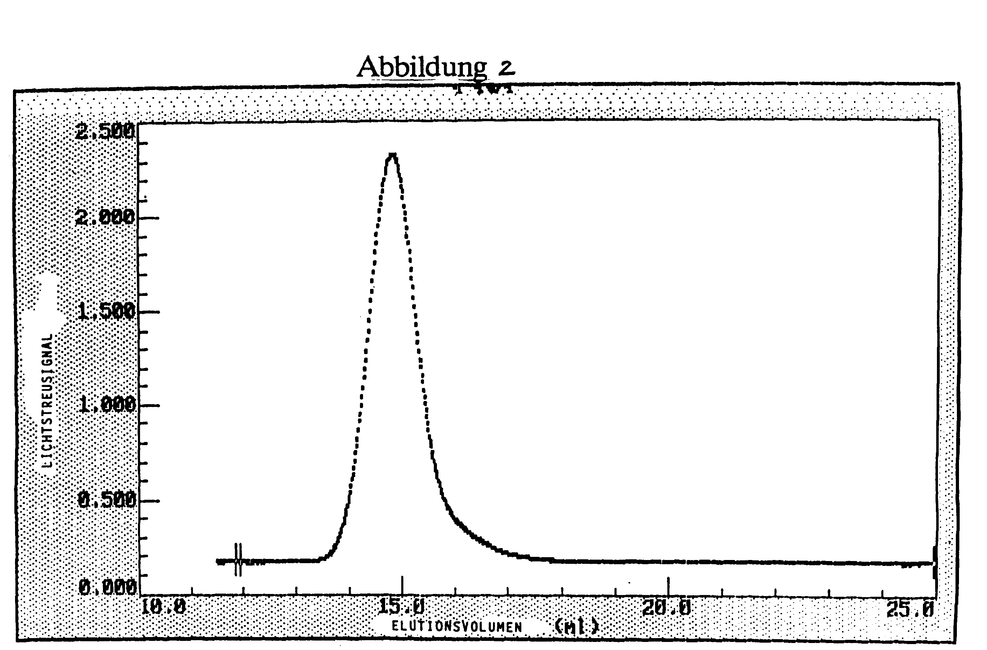

In den Figuren 1 und 2 ist die Wirkung der Hochdruckhomogenisation zur Herstellung von HES 450 (mittleres Molekulargewicht = 450.000 Dalton) gezeigt. Dabei ist in Figur 1 die Abnahme des mittleren Molekulargewichts in Abhängigkeit von der Anzahl der Durchgänge dargestellt, während Figur 2 die Molekulargewichtsverteilung bei der Ausschlußchromatographie mittels Lichtstreuung über das Elutionsvolumen (Gewichtsmittel) zeigt.

Das nachfolgende Beispiel erläutert die Erfindung.

Teilabgebaute Wachsmaisstärke mit einem mittleren Molekulargewicht von 2.689.000 Dalton wurde in an sich bekannter Weise mit Ethylenoxid zu HES umgesetzt. Eine 15 Gew.-%ige Lösung dieses HES-Produkts in ungereinigter Form wurde bei Temperaturen von etwa 50 - 70°C und einem Druck von etwa 1600 bar insgesamt 10 mal in einem Hochdruckhomogenisator der Fa. APV-Gaulin, Lübeck homogenisiert. Nach 10 Durchgängen wurde ein Molekulargewicht (Gewichtsmittel M_{w}) von etwa 670.300 Dalton gefunden, wie dies in Abb. 1 veranschaulicht ist.

## Patentansprüche

1. Verfahren zur Herstellung von Stärkeabbauprodukten mit enger Molekulargewichtsverteilung, dadurch gekennzeichnet, daß man in Abwesenheit von die Hydrolyse beschleunigenden Enzymen Stärke oder Stärkederivate in wässriger Dispersion, Suspension oder Lösung der Hochdruckhomogenisierung im Temperaturbereich von 5 - 95°C bei einem Druck von 500 bis 2000 bar und mehr unterwirft.

2. Verfahren nach Anspruch 1, dadurch gekennzeichnet, daß man die Hochdruckhomogenisierung so oft durchführt, bis das gewünschte mittlere Molekulargewicht M_{w} erreicht ist.

3. Verfahren nach Anspruch 1 oder 2, dadurch gekennzeichnet, daß man als Stärkederivat teilhydrolysierte Stärke, die durch Säurehydrolyse und/oder Enzymhydrolyse erhalten wurde, vorzugsweise mit einem mittleren Molekulargewicht von > 200 000 Dalton einsetzt.

4. Verfahren nach Anspruch 1, dadurch gekennzeichnet, daß man als Stärke native Stärke, vorwiegend Amylopektin einsetzt.

5. Verfahren nach Anspruch 1 oder 4, dadurch gekennzeichnet, daß man eine durch Verkleisterung hergestellte wässrige Dispersion einer nativen Stärke, vorzugsweise in einer Menge von 5 - 60 Gew.-% einsetzt.

6. Verfahren nach einem der Ansprüche 1 bis 3, dadurch gekennzeichnet, daß man eine 5 - 40 Gew.-%ige wässrige Dispersion einer teilhydrolysierten Stärke oder eines teilhydrolysierten Stärkederivats einsetzt.

7. Verfahren nach einem der Ansprüche 1 bis 3 und 6, dadurch gekennzeichnet, daß man die nach der Hydrolyse oder Veretherung erhaltene Reaktionsmischung ohne vorherige Isolierung des Reaktionsprodukts der Hochdruckhomogenisierung unterwirft.

8. Verfahren nach einem der Ansprüche 1 bis 7, dadurch gekennzeichnet, daß man die Hochdruckhomogenisierung bei Raumtemperatur durchführt.

## Claims

1. A process for the production of starch decomposition products with narrow molecular weight distribution, characterised in that, in the absence of hydrolysis-accelerating enzymes, starch or starch derivatives are subjected to high-pressure homogenization in aqueous dispersion, suspension, or solution, in the temperature range from 5-95°C at a pressure from 500 to 2000 bar and more.

2. A process according to Claim 1, characterised in that the high-pressure homogenisation is carried out sufficiently often until the desired mean molecular weight M_{w} is attained.

3. A process according to Claims 1 or 2, characterised in that, as a starch derivative, partially-hydrolysed starches, which were obtained by acid hydrolysis and/or enzyme hydrolysis, are used, for preference with a mean molecular weight of > 200 000 Dalton.

4. A process according to Claim 1, characterised in that native starch, and for preference amylopectin, is used as the starch.

5. A process according to Claim 1 or 4, characterised in that an aqueous dispersion of a native starch is used, produced by gelatinization, for preference in a quantity of 5-60 percent by weight.

6. A process according to one of Claims 1 to 3, characterised in that an aqueous dispersion of 5-40 percent by weight of a partially-hydrolysed starch derivative is used.

7. A process according to one of Clairns 1 to 3 and 6, characterised in that the reaction mixture obtained after the hydrolysis or etherisation, is subjected to the high-pressure homogenization without prior isolation of the reaction product.

8. A process according to one of Claims 1 to 7, characterised in that high-pressure homogenization at ambient temperature.

## Revendications

1. Procédé pour la préparation de produits de décomposition de l'amidon présentant une répartition étroite du poids moléculaire, caractérisé en ce qu'on soumet, en l'absence d'enzymes accélérant l'hydrolyse, de l'amidon ou des dérivés de l'amidon en dispersion, en suspension ou en solution aqueuse à une homogénéisation sous haute pression dans le domaine de température de 5 à 95°C, sous une pression de 500 à 2000 bar et plus.

2. Procédé selon la revendication 1, caractérisé en ce qu'on répète l'homogénéisation sous haute pression jusqu'à ce que l'on obtienne le poids moléculaire moyen désiré M_{w}.

3. Procédé selon la revendication 1 ou 2, caractérisé en ce qu'on met en oeuvre, à titre de dérivé de l'amidon, de l'amidon partiellement hydrolysé que l'on a obtenu par hydrolyse acide et/ou par hydrolyse enzymatique, de préférence possédant un poids moléculaire moyen > 200.000 dalton.

4. Procédé selon la revendication 1, caractérisé en ce qu'on met en oeuvre, à titre d'amidon, de l'amidon naturel, principalement de l'amylopectine.

5. Procédé selon la revendication 1 ou 4, caractérisé en ce qu'on met en oeuvre une dispersion aqueuse d'un amidon naturel, préparée par transformation en pâte, de préférence en une quantité de 5-60% en poids.

6. Procédé selon l'une quelconque des revendications 1 à 3, caractérisé en ce au'on met en oeuvre une dispersion aqueuse à concurrence de 5 à 40% en poids d'un amidon partiellement hydrolysé ou d'un dérivé de l'amidon partiellement hydrolysé.

7. Procédé selon l'une quelconque des revendications 1 à 3 et 6, caractérisé en ce qu'on soumet à l'homogénéisation sous haute pression le mélange réactionnel obtenu après l'hydrolyse ou l'éthérification, sans isolation préalable du produit réactionnel.

8. Procédé selon l'une quelconque des revendications 1 à 7, caractérisé en ce qu'on effectue l'homogénéisation sous haute pression à la température ambiante.
